# EUROPEAN PATENT APPLICATION

(11) **EP 3 862 349 A1**
(43) Date of publication of application: **11.08.2021**
(21) Application number: 20156456.4
(22) Date of filing: 10.02.2020
(51) Int. Cl.: C07D 401/14, C30B 29/16, C30B 29/54, G01N 21/21, G01N 23/20, G01N 23/20008

(54) **HYDRATED CRYSTALLINE POLYNUCLEAR METAL COMPLEX, HYDRATED CRYSTALLINE POLYNUCLEAR METAL COMPLEX INCLUDING A GUEST COMPOUND ANALYTE AND ITS USE IN A METHOD FOR DETERMINING MOLECULAR STRUCTURE OF THE GUEST COMPOUND ANALYTE**

(71) Applicant: Merck Patent GmbH, 64293 Darmstadt (DE)
(72) Inventor: KUEHN, Clemens, 64293 Darmstadt (DE)
(74) Representative: Merck Serono S.A. Intellectual Property

(57) **Abstract**

The present invention is directed to hydrated crystalline polynuclear metal complexes. These hydrated crystalline metal complexes of the present invention can absorb an analyte guest compound to form a crystal structure analysis sample. The molecular structure of the analyte guest compound can be determined by X-ray crystallography using the crystal structure analysis sample obtained with the hydrated crystalline polynuclear metal complexes.

## Description

### Field of the Invention

The present invention relates to crystalline polynuclear metal complexes and particularly those crystalline polynuclear metal complexes which include a guest compound analyte. Such crystalline polynuclear complexes are useful in crystal structure analysis methods to determine the molecular structure of the guest compound analyte present in the crystalline polynuclear metal complex. The present invention also relates to the use of such crystalline polynuclear complexes in methods for determining the molecular structure of a guest compound analyte through crystal structure analysis.

### Background

X-ray single crystal structure analysis has been known as a method for determining the molecular structure of an organic compound. The molecular structure of an organic compound can be accurately determined using X-ray single crystal structure analysis when it is possible to prepare a high-quality single crystal.

However, when the amount of organic compound is very small, and it is impossible to obtain a sufficient amount of single crystal, it is difficult to employ X-ray single crystal structure analysis for determining the molecular structure of the organic compound. It is difficult to prepare a single crystal when the organic compound for which the molecular structure is to be determined is liquid at about room temperature (i.e., when the melting point of the organic compound is equal to or lower than room temperature). This difficulty may be for example as a result of the inability of the compound in forming crystals at room temperature. Also, the difficulty can simply be that it is extremely difficult to obtain sufficient quantities of appropriate purity of the compound analyte. The use of a crystalline polynuclear complex, which is porous, acts as a framework to support the compound analyte in an ordered manner.

The use of crystalline polynuclear metal complexes that can incorporate a guest compound analyte in an ordered manner has enabled the use of X-ray single crystal structure analysis even in instances wherein it is difficult to obtain a sufficient amount of a single crystal of the analyte. Such method of determining the molecular structure of the analyte compound is known as the crystalline sponge method. This emerging technology in X-ray crystallography makes it possible to obtain the crystal structure of non-crystalline (or hard-to-crystallize) compounds, without crystallization, on a microgram scale. The use of crystalline polynuclear metal complexes or crystalline sponges for facilitated structure elucidation of small molecules is described in for example WO 2014/038220 and WO 2016/143872 and in for example Y. Inokuma et al., Nature 2013, 495, 461-466 (Corrigendum: Nature 2013, 501) and M. Hoshino, et al., IUCrJ, 2016, 3, 139-151. This method uses a porous crystalline polynuclear metal complex, for example [(ZnX₂)₃•(tpt)₂]ₙ (wherein X = Cl or I; and tpt = 2,4,6-tris(4-pyridyl)-1,3,5-triazine) as a crystalline sponge that can absorb small to medium sized organic molecules, orient them uniformly in their pores, and make them observable by conventional single crystal X-ray crystallography.

In order to prepare a crystalline polynuclear metal complex which envelops a guest compound analyte the crystalline polynuclear metal complex is first prepared in an appropriate solvent for the crystallization of the crystalline polynuclear metal complex. The obtained crystalline polynuclear metal complex can be used immediately in a subsequent step to introduce the guest compound analyte or can be stored for later use. Storage of the crystalline polynuclear metal complex is generally in an appropriate storage solvent.

Although these methods using crystalline polynuclear metal complexes have been successful under certain conditions, the quality of the crystalline polynuclear metal complexes is often insufficient for the accurate determination of the molecular structure of a guest compound analyte.

Currently, the most widely used crystalline sponge material is the chloride based crystalline polynuclear metal complex [(ZnCl₂)₃(tpt)₂]ₙ. However, the single crystals of this material are very sensitive to minute amounts of water, which is ubiquitous for example in the form of ambient humidity or present in small amounts in organic solvents. Upon contact with the crystalline polynuclear metal complex, such water molecules are rapidly incorporated into the sponge material, leading to rapid changes in the local crystal structure. These changes induce a high level of internal stress due to the misfit of compartments free of water and those containing water. These misfits in turn give rise to formation of cracks and therefore loss of single crystallinity or complete destruction of the crystals rendering such crystalline polynuclear metal complexes unusable for the crystalline sponge X-ray crystallography technology.

As a result, the crystalline sponge X-ray crystallography technology when using these crystalline polynuclear metal complexes requires strict use of freshly prepared material or storage of previously prepared crystalline material under strictly controlled dry conditions (such as dry protecting atmospheres) as well as use of dry solvents and analytes. Such requirements complicate and may limit the use of the crystalline sponge X-ray crystallography technology.

### Summary of the Invention

The current invention provides a solution to the above described limitations on the use of the crystalline polynuclear metal complexes, particularly those using zinc-halogen based crystalline polynuclear metal complex such as for example [(ZnCl₂)₃(tpt)₂]ₙ. The present invention provides for a hydrated crystalline polynuclear metal complex exhibiting the same metal-organic framework but containing certain amounts of water. The water already present in the crystal lattice reduces the hygroscopicity of the crystalline polynuclear metal complex material and therefore stabilizes against the rapid uptake of water and the subsequent destruction of the crystalline structure. The hydrated material of the present invention is usable in crystalline sponge X-ray crystallography methods and can be stored under conditions without having the precautionary methods required for conventional crystalline polynuclear metal complexes used in this technology.

In one embodiment the present invention provides a hydrated crystalline polynuclear metal complex, wherein the hydrated polynuclear metal complex is represented by [[M(X)₂]₃(L)₂]ₙ · (H₂O)ₚ wherein
M is a metal ion,
X is a monovalent anion,
L is a tridentate ligand represented by formula (1), 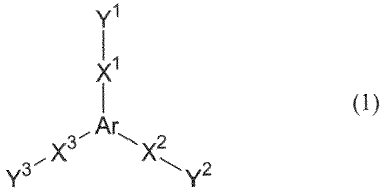 wherein Ar is a substituted or unsubstituted trivalent aromatic group, X¹ to X³ are independently a divalent organic group, or a single bond that directly bonds Ar and Y¹, Y², or Y³, and Y¹ to Y³ are independently a monovalent organic group having a coordinating moiety,
n is an arbitrary natural number, and
p is an arbitrary number,
wherein the amount of hydration of the polynuclear metal complex represented by the value p is such that p has a value between 0.5 to 3 and wherein water of hydration is hydrogen bonded to the polynuclear metal complex.

In another embodiment there is provided a method of preparing a hydrated crystalline polynuclear metal complex comprising the steps of:
a) layering a volume of a lower alcohol, for example methanol, onto a solution of ligand L in a mixture of an organic (nonpolar) solvent, for example CHCl₃, cyclohexane or n-hexane, and the lower alcohol, for example methanol,
b) layering onto the volume of the lower alcohol of step a) a solution of MX₂ in a mixture of the lower alcohol and water,
c) obtaining a crystalline material after maintaining the solution obtained in step b) for a period of 1 to 10, preferably 3 to 7 days at a temperature of 15°C to 40°C, preferably at 20°C to 30°C, and
d) washing the crystalline material of step c) with an organic (nonpolar) solvent, for example with CHCl₃ or cyclohexane or n-hexane.
As used herein the term lower alcohol refers to an alcohol with 1 to 6, preferably 1 to 4 carbon atoms.

In yet another embodiment there is provided method of producing a crystal structure analysis sample, comprising bringing a hydrated crystalline polynuclear metal complex of the present invention into contact with an analysis target compound (analyte) containing solution to arrange molecules of an analyte in the pores and/or voids of the hydrated crystalline polynuclear metal complex in an ordered manner.

In another embodiment of the present invention there is provided a method for determining a molecular structure of an analysis target compound, comprising performing crystal structure analysis using a crystal structure analysis sample obtained by a method for producing the crystal structure analysis sample using a hydrated crystalline polynuclear metal complex of the present invention.

### Brief Description of the Drawings

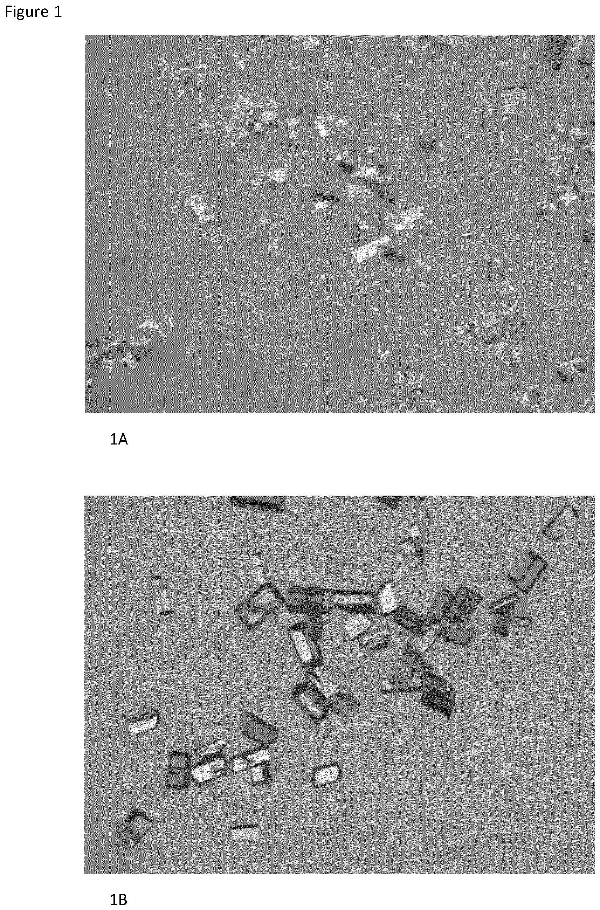

Figure 1: Shows bulk crystalline polynuclear metal complex material in its unhydrated form and its hydrated form after storage for 51 days at 22°C and 73% relative humidity. In figure 1A is shown bulk unhydrated crystalline [(ZnCl₂)₃(tpt)₂]ₙ. Figure 1B shows bulk hydrated crystalline [(ZnCl₂)₃(tpt)₂]ₙ · (H₂O)_{1.5}. The pictures in both figure 1A and 1B are at a 50-fold magnification.

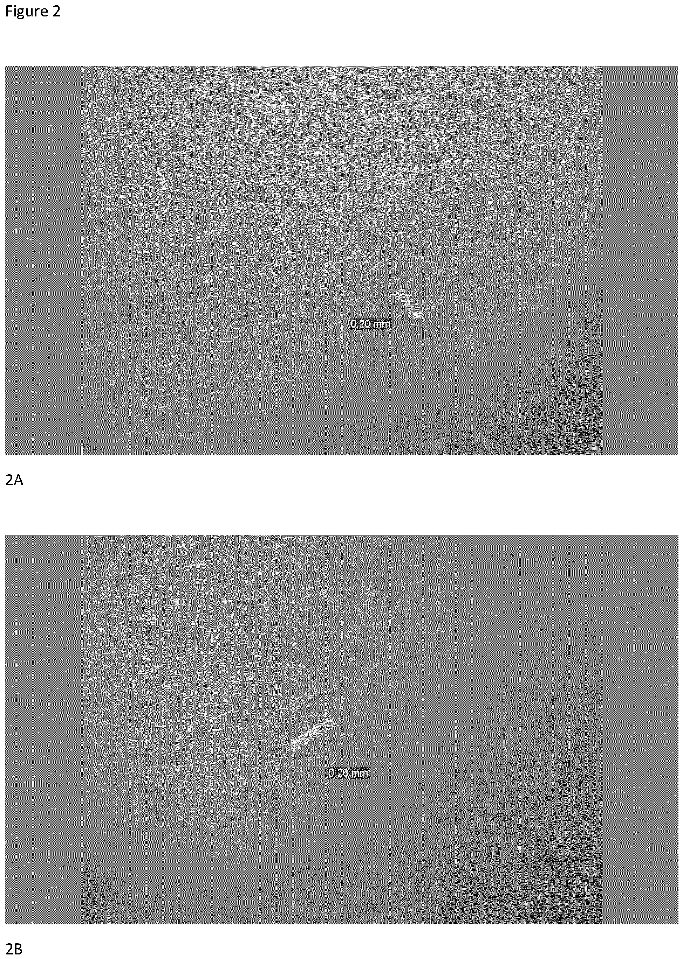

Figure 2: Shows an individual crystal of unhydrated and hydrated crystalline polynuclear metal complex after storage for 51 days at 22°C and 73% relative humidity. In figure 2A an unhydrated [(ZnCl₂)₃(tpt)₂]ₙ is shown whereas in figure 2B a hydrated [(ZnCl₂)₃(tpt)₂]ₙ · (H₂O)_{1.5} is shown. The pictures in both figure 2A and 2B are both at a 50-fold magnification.

### Detailed Description of the Invention

A hydrated crystalline polynuclear metal complex and its use in single crystal X-ray crystallography for the elucidation of the molecular structure of an analyte guest compound, as well as methods of preparing such hydrated crystalline polynuclear metal complex are described in detail below.

The hydrated crystalline polynuclear metal complex used in connection with the embodiments of the invention has a three-dimensional network structure that includes a ligand having coordinating moieties, and a metal ion that serves as a center metal. The term "three-dimensional network structure" used herein refers to a network-like structure in which a structural unit formed by a ligand (i.e., a ligand having two or more coordinating moieties and an additional monodentate ligand) and a metal ion that is bonded to the ligand is repeatedly arranged three-dimensionally.

The three-dimensional network structure results in the hydrated crystalline polynuclear metal complex of the present invention to have a porous structure. Such porous structure enables the capturing of an analyte guest compound within the porous structure of the hydrated crystalline polynuclear metal complex. As such the crystalline polynuclear metal complex acts as a "sponge" for the analyte guest compound. Introduction of such analyte guest compound in a hydrated crystalline complex of the present invention allows for the organized arrangement of the analyte guest compound within the complex molecule. This organized arrangement of the analyte guest compound enables the use of single crystal X-ray crystallography to elucidate the molecular structure of the analyte guest compound. Therefore, the single crystal X-ray crystallography method using such crystalline polynuclear metal complexes is often referred to as the Crystalline Sponge (CS) X-ray crystallography method.

Any disruption of the organized porous structure of the crystalline polynuclear metal complex (crystalline sponge) has a detrimental effect on the usability of the crystalline polynuclear metal complex as a support structure ("sponge structure") for the analyte guest compound in single crystal X-ray crystallography. Water, even in minute amounts, can have such destructive effect on the crystallinity of the crystalline polynuclear metal complexes. Water is ubiquitous for example in the form of ambient humidity or present in small amounts in organic solvents. Upon contact with the crystalline polynuclear metal complex, such water molecules will be rapidly incorporated into the sponge material, leading to rapid changes in the local crystal structure giving rise to fractures and loss of single crystallinity or even break-up of the three-dimensional network structure. The present invention described herein is directed to hydrated crystalline polynuclear metal complexes which are much less susceptible or sensitive to small amounts of water in the environment.

The hydrated crystalline polynuclear metal complex includes a coordinating metal complex is represented by [[M(X)₂]₃(L)₂]ₙ · (H₂O)ₚ wherein
M is a metal ion,
X is a monovalent anion,
L is a tridentate ligand represented by formula (1), 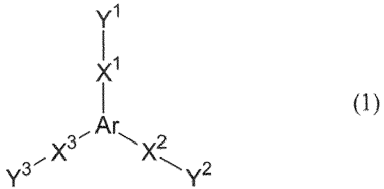 wherein Ar is a substituted or unsubstituted trivalent aromatic group, X¹ to X³ are independently a divalent organic group, or a single bond that directly bonds Ar and Y¹, Y², or Y³, and Y¹ to Y³ are independently a monovalent organic group having a coordinating moiety,
n is an arbitrary natural number, and
p is an arbitrary number,
wherein the amount of hydration of the polynuclear metal complex represented by the value p is such that p has a value between 0.5 to 3 and wherein water of hydration is hydrogen bonded to the polynuclear metal complex.

The tridentate aromatic ligand Ar in the formula (1) is a trivalent aromatic group. The number of carbon atoms of Ar is normally 3 to 22, preferably 3 to 13, and more preferably 3 to 6. Examples of Ar include a trivalent aromatic group having a monocyclic structure that consists of one 6-membered aromatic ring, and a trivalent aromatic group having a fused ring structure in which three 6-membered aromatic rings are fused.

Examples of the trivalent aromatic group having a monocyclic structure that consists of one 6-membered aromatic ring include the groups respectively represented by the following formulas (2a) to (2d). Examples of the trivalent aromatic group having a fused ring structure in which three 6-membered aromatic rings are fused, include the group represented by the following formula (2e). It is noted that "*" in the formulas (2a) to (2e) indicates the positions at which X¹ to X³ are bonded.

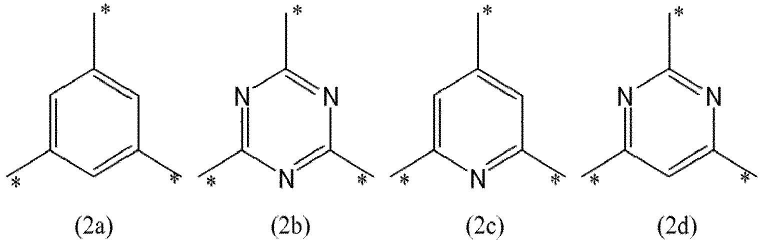

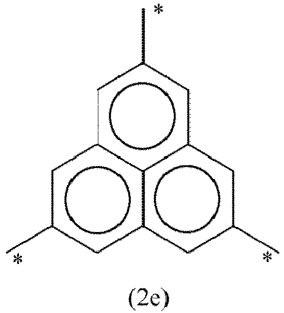

The aromatic groups represented by the formulas (2a) and (2c) to (2e) may be substituted with a substituent at an arbitrary position. Examples of a substituent include an alkyl group such as a methyl group, an ethyl group, an isopropyl group, an n-propyl group, and at-butyl group; an alkoxy group such as a methoxy group, an ethoxy group, an n-propoxy group, and an n-butoxy group; a halogen atom such as a fluorine atom, a chlorine atom, and a bromine atom; and the like. Ar is preferably the aromatic group represented by the formula (2a) or (2b), and particularly preferably the aromatic group represented by the formula (2b).

X¹ to X³ are independently a divalent organic group, or a single bond that directly bonds Ar and Y¹, Y², or Y³. The divalent organic group that may be represented by X¹ to X³ is preferably a group that can form a pi electron conjugated system together with Ar. When the divalent organic group that may be represented by X¹ to X³ forms a pi electron conjugated system, the planarity of the tridentate ligand represented by the formula (1) is improved, and a strong three-dimensional network structure is easily formed. The number of carbon atoms of the divalent organic group is preferably 2 to 18, more preferably 2 to 12, and still more preferably 2 to 6.

Y¹ to Y³ are independently a monovalent organic group having a coordinating moiety. The organic group represented by Y¹ to Y³ is preferably a group that can form a pi electron conjugated system together with Ar and X¹ to X³. When the organic group represented by Y¹ to Y³ forms a pi electron conjugated system, the planarity of the tridentate ligand represented by the formula (1) is improved, and a strong three-dimensional network structure is easily formed.

The number of carbon atoms of the organic group represented by Y¹ to Y³ is preferably 5 to 11, and more preferably 5 to 7. Examples of the organic group represented by Y¹ to Y³ include the organic groups respectively represented by the following formulas (3a) to (3f). It is noted that "*" in the formulas (3a) to (3f) indicates the position at which X¹, X², or X³ is bonded.

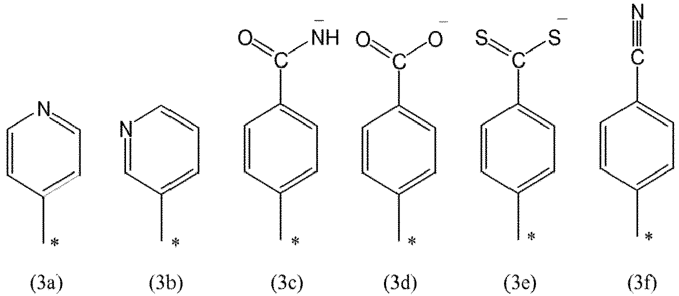

The organic groups represented by the formulas (3a) to (3f) may be substituted with a substituent at an arbitrary position. Examples of the substituent include those mentioned above in connection with Ar. The group represented by the formula (3a) is particularly preferable as Y¹ to Y³.

The size of the pores and the like of the polynuclear-metal complex can be adjusted by appropriately selecting Ar, X¹ to X³, and Y¹ to Y³ in the tridentate ligand represented by the formula (1). The method according to one embodiment of the invention makes it possible to efficiently obtain a stable single crystal of a polynuclear metal complex that has pores and the like having a size sufficient to include an organic compound for which the molecular structure is to be determined.

It is preferable that the tridentate ligand represented by the formula (1) have high planarity and high symmetry and have a structure in which a pi-conjugated system extends over the entire ligand, since a strong three-dimensional network structure is easily formed. Examples of such a tridentate ligand include the ligands respectively represented by the following formulas (4a) to (4f).

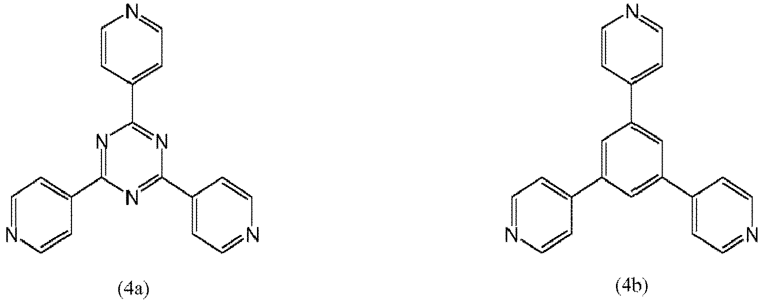

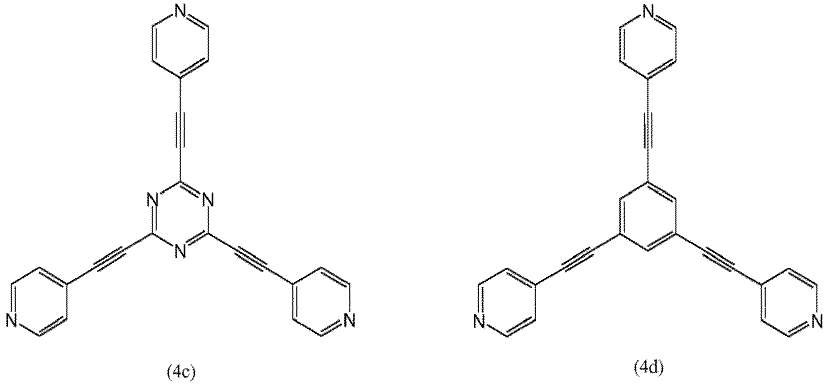

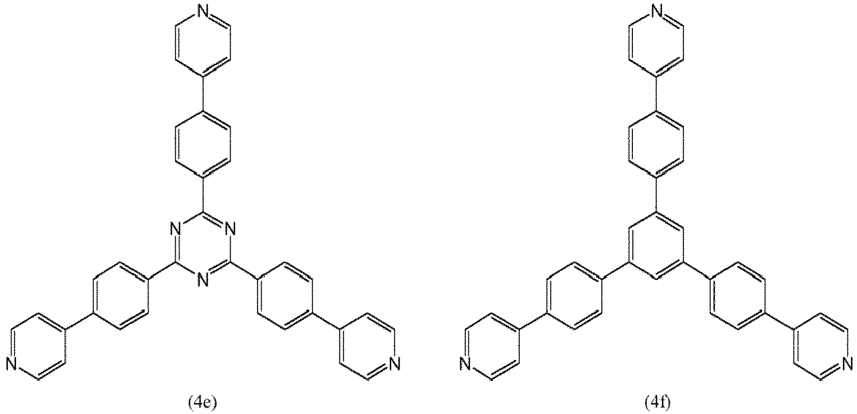

Among these, 2,4,6-tris(4-pyridyl)-1,3,5-triazine (TPT) represented by the formula (4a) is particularly preferable as the tridentate ligand represented by the formula (1).

The metal ion that serves as the center metal is not particularly limited as long as the metal ion forms a coordination bond together with the multidentate ligand to form the three-dimensional network structure. A known metal ion may be used as the metal ion that serves as the center metal. It is preferable to use an ion of a metal among the metals that belong to Groups 8 to 12 in the periodic table, such as an iron ion, a cobalt ion, a nickel ion, a copper ion, a zinc ion, or a silver ion, and more preferably an ion of a divalent metal among the metals that belong to Groups 8 to 12 in the periodic table. It is particularly preferable to use a zinc(II) ion or a cobalt(II) ion, since a polynuclear-metal complex having large pores and the like can be easily obtained. Even more preferably is the use of zinc(II) ion.

The crystalline polynuclear metal complex used in connection with the embodiments of the invention is normally stabilized due to coordination of a monodentate ligand that serves as a counter ion in addition to the neutral multidentate ligand. Examples of the monodentate ligand include a monovalent anion such as a halogen ion such as a chloride ion (Cl⁻), a bromide ion (Br⁻) and an iodide ion (I⁻), and a thiocyanate ion (SCN⁻). Preferably the monovalent anion is a chloride ion (Cl⁻).

The polynuclear-metal complex used in connection with the embodiments of the invention may include a solvent. Such solvent in most instances having only a weak association with the crystalline polynuclear metal complex and can easily being exchanged with another solvent. The solvent present in the complex is often result of the method of producing the complex. Frequently for storage purposes the solvent is chloroform (CHCl₃) but any other suitable storage solvent may be used in which the crystalline polynuclear metal complex remains intact, for example cyclohexane or n-hexane. The solvent may be exchanged prior to use in a process for introducing an analyte guest compound into the complex. Particularly when the crystalline polynuclear metal complex has been stored in chloroform and the solvent used in the process to introduce the analyte guest compound is not chloroform.

The crystalline polynuclear metal complex used in connection with the embodiments of the invention has the three-dimensional network structure that is formed by the metal ion and the tridentate ligand that is coordinated to the metal ion, and has pores and the like which may be three-dimensionally arranged in the three-dimensional network structure in an ordered manner.

The expression "the pores and the like that are three-dimensionally arranged in the three-dimensional network structure in an ordered manner" means that the pores and the like are arranged in the three-dimensional network structure in an ordered manner to such an extent that the pores and the like can be observed by X-ray single crystal structure analysis. The three-dimensional network structure is not particularly limited as long as the three-dimensional network structure has the above structural features, and the pores and the like have a size sufficient to include the analyte guest compound, often an organic compound, for which the molecular structure is to be determined.

To reduce or ameliorate the deleterious effect of water on the crystalline polynuclear metal complex, the crystalline polynuclear metal complex of the present invention also includes water of hydration into the crystalline polynuclear metal complex thereby forming a hydrated crystalline polynuclear metal complex of the present invention. The water of hydration is hydrogen bonded to the crystalline polynuclear metal complex. The hydration of the crystalline polynuclear metal complex is from 0.5 to 3 molecules of water per unit of the polynuclear metal complex. Preferable the amount of water is between 0.5 and 2 molecules of water, even more preferably between 0.5 and 1.5 molecules of water, even more preferably the amount of water is between 1 and 1.5 molecules of water per unit of the polynuclear metal complex.

The hydrated crystalline polynuclear metal complex has increased stability upon storage. Storage under ambient conditions would not lead to fractures and loss of single crystallinity or even break-up of the three-dimensional network structure of the crystalline material which is observed with unhydrated crystalline material. In addition, the hydrated crystalline material can be produced with higher yields and increased efficiency. For example, using a method of preparation of such hydrated crystalline material as in one embodiment of the current invention yields bulk material of higher quality. The amount of usable crystals of the polynuclear metal complex, i.e. crystals of appropriate size of between 100 to 300 µm and uniformity, is much higher in material produced according to a method as in the current invention. As a result of these advantages, the hydrated crystalline polynuclear metal complex material can be produced more economically and can be stored and transported without the need to maintain special storage conditions which may be expensive and/or impractical.

In one embodiment the hydrated crystalline polynuclear metal complex is produced by a method comprising the steps of: a) layering a volume of a lower alcohol such as for example methanol onto a solution of ligand L in a mixture of an organic (nonpolar) solvent, for example CHCl₃ or cyclohexane or n-hexane, and the lower alcohol such as for example methanol, b) layering onto the volume of the lower alcohol of step a) a solution of MX₂ in a mixture of the lower alcohol and water, c) obtaining a crystalline material after maintaining the solution obtained in step b) for a period of about 1 to 10 days, preferably of about 3 to 7 days, at a temperature of about 15°C to 40°C, preferably at about 20°C to 30°C, and d) washing the crystalline material of step c) with an organic (nonpolar) solvent, for example CHCl₃ or cyclohexane or n-hexane. As used herein the term lower alcohol refers to an alcohol with 1 to 6, preferably 1 to 4 carbon atoms. In a preferred embodiment the lower alcohol is methanol or ethanol, even more preferred is wherein the lower alcohol is methanol. Preferably the organic (nonpolar) solvent as used herein is a lipophilic solvent such as for example CHCl₃, cyclohexane or n-hexane.

In a preferred embodiment of the present invention, the hydrated crystalline polynuclear metal complex includes Zn(ll) as the metal ion. In such preferred embodiment the monovalent anion is either chloride (Cl⁻) or Iodide (I⁻). In such hydrated crystalline polynuclear metal complex the preferred ligand L is 2,4,6-tris(4-pyridyl)-1,3,5-triazine (TPT). As such in hydrated form the crystalline polynuclear metal complex in a preferred embodiment is represented by [(ZnX₂)₃-(tpt)₂]ₙ · (H₂O)ₚ, wherein X is a halogen selected from chloride (Cl), iodide (I) and bromide (Br), n is an arbitrary natural number and p is an arbitrary number between 0.5 and 3. Preferably, X is a halogen selected from chloride (CI) and iodide (I). More preferably the hydrated polynuclear metal complex is [(ZnCl₂)₃(tpt)₂]ₙ · (H₂O)ₚ. Even more preferably the hydrated crystalline polynuclear metal complex is [(ZnCl₂)₃(tpt)₂]ₙ · (H₂O)_{1.5}. In embodiments wherein X is a bromide (Br) the solution of ligand L does not contain nitrobenzene.

The hydrated crystalline polynuclear metal complexes of the present invention can be used in any known method for use in the sponge method of single crystal X-ray crystallography to determine the molecular structure of an analysis target compound. In such sponge method an analysis target compound is first introduced into the hydrated crystalline polynuclear metal complex of the present invention to obtain a crystal structure analysis sample. Such crystal structure analysis sample may be obtained by a method comprising bringing the hydrated single crystal polynuclear metal complex of the present invention into contact with an analysis target compound (analyte) containing solution to absorb molecules of an analyte in the pores and/or voids of the hydrated single crystal polynuclear metal complex. Considering that the pores in the hydrate crystalline polynuclear metal complex of the present invention are regularly and ordered such analyte is absorbed in an ordered manner in order to be useful a method to determine the molecular structure by X-ray crystallography.

As such, in another embodiment of the present invention there is provided a method for determining a molecular structure of an analysis target compound, comprising performing crystal structure analysis using a crystal structure analysis sample obtained by such method for producing the crystal structure analysis sample as in the present invention.

### Examples

### Example 1: Crystallization of ZnCl₂-tpt hydrate.

21.27 mg tris(4-pyridyl)-1,3,5-triazine (tpt) is dissolved in a mixture of 16 mL CHCl₃ and 0.833 mL methanol. 18.42 mg of ZnCl₂ are dissolved in a mixture of 2 mL methanol and 300 µL water. A test tube is filled with 5 mL of the tpt solution. 400 µL MeOH is carefully layered on top of the tpt solution. Finally, 600 µL of a ZnCl₂ solution is carefully layered on top of the methanol layer while avoiding instant mixing of the solutions. The so prepared layered solutions are kept in a closed environment at ambient temperature for ca. 7 days.

After seven days, crystals of block to short needle shape and a size of 100 - 300 µm are harvested and stored in CHCl₃.

### Example 2: Crystallization of ZnCl₂-tpt (unhydrated).

The unhydrated crystalline ZnCl₂-tpt polynuclear metal complex is prepared as in example 1. However, the ZnCl₂ solution as described in example 1 is prepared as follows. 18.42 mg of ZnCl₂ are dissolved in 2.3 mL methanol. As before, after a period of 7 days for crystallization, crystals of block to short needle shape and a size of 100 - 300 µm are harvested and stored in CHCl₃.

### Example 3: Stability assay for crystalline ZnCl₂-tpt polynuclear metal complexes.

Samples of both the crystalline ZnCl₂-tpt hydrated and unhydrated material as prepared according to examples 1 and 2 were tested for their stability. All sample material was stored for 51 days at 22°C and at a relative humidity of 73%. After storage the material was visually inspected at a 50-fold magnification. Test samples included both bulk material as well as individual crystal material. The unhydrated crystalline ZnCl₂-tpt polynuclear metal complex bulk material resulted in a mixture of crystalline material of very small size as well as limited amounts of crystalline material of appropriate size (i.e. between 100 - 300 µm) (See figure 1A). In contrast, the hydrated bulk crystalline ZnCl₂-tpt polynuclear material shows almost exclusively block or short needle shaped crystals of appropriate size (see figure 1B). As a result, the hydrated bulk material provides much higher amount of usuable crystalline material compared to the unhydrated material. Therefore, the yield and efficiency are also much higher when producing hydrated crystalline material.

In addition, when comparing a single unhydrated crystal ZnCl₂-tpt polynuclear metal complex (as in figure 2A) with a single hydrated crystal ZnCl₂-tpt polynuclear metal complex (as in figure 2B) stored under the same conditions, it is clear that the hydrated material is significantly more stable. The unhydrated ZnCl₂-tpt polynuclear metal complex as shown in figure 2A shows deterioration, including fraction-lines and severe discoloration. In contrast, the hydrated ZnCl₂-tpt polynuclear metal complex as shown in figure 2B shows that even after storage for 51 days under the same conditions as the unhydrated material no deterioration can be observed, and the single crystal is smooth and of uniform color.

## Claims

1. A hydrated crystalline polynuclear metal complex, wherein the hydrated polynuclear metal complex is represented by [[M(X)₂]₃(L)₂]ₙ · (H₂O)ₚ wherein
M is a metal ion,
X is a monovalent anion,
L is a tridentate ligand represented by formula (1), 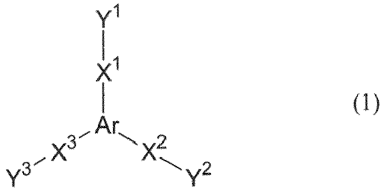 wherein Ar is a substituted or unsubstituted trivalent aromatic group, X¹ to X³ are independently a divalent organic group, or a single bond that directly bonds Ar and Y¹, V², or Y³, and Y¹ to Y³ are independently a monovalent organic group having a coordinating moiety,
n is an arbitrary natural number, and
p is an arbitrary number,
wherein the amount of hydration of the polynuclear metal complex represented by the value p is such that p has a value between 0.5 to 3 and wherein water of hydration is hydrogen bonded to the polynuclear metal complex.

2. The hydrated crystalline polynuclear metal complex of claim 1, wherein p has a value between 0.5 to 2.

3. The hydrated crystalline polynuclear metal complex of claim 2, wherein p has a value between 1 and 1.5.

4. The hydrated crystalline polynuclear metal complex of any one of the preceding claims, wherein the hydrated polynuclear metal complex is [(ZnX₂)₃(tpt)₂]ₙ · (H₂O)ₚ.

5. The hydrated crystalline polynuclear metal complex of claim 4, wherein the hydrated polynuclear metal complex is [(ZnCl₂)₃(tpt)₂]ₙ · (H₂O)ₚ.

6. The hydrated crystalline polynuclear metal complex of claim 5, wherein the hydrated polynuclear metal complex is [(ZnCl₂)₃(tpt)₂]ₙ · (H₂O)_{1.5}.

7. The hydrated crystalline polynuclear metal complex of any of the preceding claims, wherein the hydrated polynuclear metal complex has a porous structure.

8. The hydrated crystalline polynuclear metal complex of any of the preceding claims, wherein X is a halogen selected from the group consisting of CI, and I.

9. The hydrated crystalline polynuclear metal complex of any one of the preceding claims, wherein the hydrated polynuclear complex is obtained by a method comprising the steps of:
a) layering a volume of a lower alcohol onto a solution of ligand L in a mixture of an organic (nonpolar) solvent and the lower alcohol,
b) layering onto the volume of methanol of step a) a solution of MX₂ in a mixture of the lower alcohol and water,
c) obtaining a crystalline material after maintaining the solution obtained in step b) for a period of 1 to 10 days, preferably 3 to 7 days, at a temperature of 15°C to 40°C, preferably at 20°C to 30°C, and
d) washing the crystalline material of step c) with an organic (nonpolar) solvent.

10. The hydrated crystalline polynuclear metal complex of claim 7, wherein when X is Br the solution of ligand L does not contain nitrobenzene.

11. The hydrated crystalline polynuclear metal complex of any one of claims 9 or 10, wherein the ligand L is 2,4,6-tris(4-pyridyl)-1,3,5-triazine (tpt) and MX₂ is ZnCl₂.

12. The hydrated crystalline polynuclear metal complex of any one of claims 9 to 11, wherein the mixture of lower alcohol/water is a mixture of methanol and water at a volume ratio of 20:3.

13. The hydrated crystalline polynuclear metal complex of any one of claims 9 to 12, wherein the mixture of the organic (nonpolar) solvent and lower alcohol is a mixture of CHCl₃ and methanol at a volume ratio of 19:1.

14. The hydrated crystalline polynuclear metal complex of any one of claims 9 to 13, wherein the volume ratio of the solution of ligand L in a mixture of CHCl₃/methanol to the volume of methanol to the solution of MX₂ in a mixture of methanol/water is 25 : 2 : 3.

15. A method of producing a crystal structure analysis sample, comprising bringing the hydrated single crystal polynuclear metal complex according to any one of claims 1 to 14 into contact with an analysis target compound (analyte) containing solution to arrange molecules of an analyte in the pores and/or voids of the hydrated single crystal polynuclear metal complex in an ordered manner.

16. A method for determining a molecular structure of an analysis target compound, comprising performing crystal structure analysis using a crystal structure analysis sample obtained by a method for producing the crystal structure analysis sample according to claim 15.

17. A method of preparing a hydrated crystalline polynuclear metal complex comprising the steps of:
a) layering a volume of methanol onto a solution of ligand L in a mixture of an organic (nonpolar) solvent and a lower alcohol,
b) layering onto the volume of the lower alcohol of step a) a solution of MX₂ in a mixture of the lower alcohol and water,
c) obtaining a crystalline material after maintaining the solution obtained in step b) for a period of 1 to 10 days, preferably 3 to 7 days at a temperature of 15°C to 40°C, preferably at 20°C to 30°C, and
d) washing the crystalline material of step c) with an organic (nonpolar) solvent.
